# EUROPEAN PATENT APPLICATION

(11) **EP 3 305 318 A1**
(43) Date of publication of application: **11.04.2018**
(21) Application number: 16192366.9
(22) Date of filing: 05.10.2016
(51) Int. Cl.: A61K 39/00, A61K 31/4745, A61K 31/513, A61K 31/519, A61P 35/00

(54) **[6R]-5,10-METHYLENETETRAHYDROFOLATE IN 5-FLUOROURACIL BASED CHEMOTHERAPY**

(71) Applicant: Isofol Medical AB, 413 46 Göteborg (SE)
(72) Inventor: GUSTAVSSON, Bengt, 42679 Västra Frölunda (SE); LINDBERG, Per Lennart, 41301 Göteborg (SE); SUNDÉN, Gunnel Elisabeth, 41301 Göteborg (SE)
(74) Representative: Awapatent A/S

(57) **Abstract**

The present invention relates to the treatment of solid tumors in humans such as cancer, especially colorectal cancer (CRC), which involves administering the diastereomerically pure folate adjuvant [6R]-5,10-methylenetetrahydrofolate in 5-fluorouracil (5-FU) based chemotherapy.

## Description

### Field

The present invention relates to the treatment of solid tumors in humans such as cancer, which involves [6R]-5,10-methylenetetrahydrofolate in 5-fluorouracil (5-FU) based chemotherapy.

### Background of the Invention

5-fluorouracil (5-FU) was first introduced in 1957, and still remains an essential part of the treatment of a wide range of solid tumours such as breast tumours, tumours of head and neck and gastrointestinal tumours.

5-FU is an example of a rationally designed anticancer agent. Observations of utilization of uracil in rat liver tumours indicated that the utilization of this nucleobase (there are four nucleobases in the nucleic acid of RNA) [Berg JM; Tymoczko JL; Stryer L (2002). Biochemistry (5th ed.), WH Freeman and Company. pp. 118-19, 781-808. ISBN 0-7167-4684-0. OCLC 179705944] was more pronounced in the tumours than in non-malignant tissue. This implicated that the enzymatic pathways for uracil utilization differs between malignant and normal cells [Rutman RJ et al. Studies in 2-acetylaminofluorene carcinogenesis. III. The utilization of uracil 2-14C by preneoplastic rat liver and rat hepatoma. Cancer Res 1954; 14: 119-123]. 5-FU was then synthesized as an antimetabolic agent [Heidelberger C et al. Fluorinated pyrimidines, a new class of tumour-inhibitory compounds. Nature 1967; 179: 663-666]. In 5-FU, the hydrogen atom in position 5 of uracil is replaced by the similar sized atom of fluorine, and 5-FU was designed to occupy the active sites of enzymes, blocking the metabolism of malignant cells.

The overall response rate of 5-FU alone is quite limited, reaching levels of 10-15 % [Johnston P.G., Kaye S. Capcetabine; a novel agent for the treatment of solid tumours. Anticancer Drugs 2001, 12: 639-646] and modulation strategies to increase the anticancer activity of 5-FU have been developed. One of the most widely used strategies is a coadministration of Leucovorin, the calcium salt of folinic acid. Leucovorin (LV) acts as a stabiliser of the ternary complex, a structure formed by ¹⁾ 5,10-methylene tetrahydrofolate, the active metabolite of LV, of ²⁾ FdUMP, the 5-FU active metabolite and of ³⁾ Thymidylate synthase. This ternary complex inhibits the enzyme thymidylate synthase, an enzyme necessary for DNA synhesis [Longley D.B. et al. 5-Fluorouracil. Mechanisms of action and clinical strategies, Nat Rev Cancer. 2003 May;3(5):330-8. Review]. By adding LV to 5-FU the overall response rates increased to over 20 % [Longley D.B. et al. 2003 *ibid*.].

Breast cancer is the most frequently diagnosed cancer and the leading cause of cancer-related death among females worldwide¹. Despite the gains in early detection, up to five percent of women diagnosed with breast cancer in the United States have metastatic disease at the time of first presentation. In addition, up to 30 percent of women with early-stage, non-metastatic breast cancer at diagnosis will develop distant metastatic disease [Early Breast Cancer Trialists' Collaborative Group (EBCTCG). Effects of chemotherapy and hormonal therapy for early breast cancer on recurrence and 15-year survival: an overview of the randomised trials. Lancet 2005; 365:1687]. Although metastatic breast cancer is not curable, meaningful improvements in survival have been seen, coincident with the introduction of newer systemic therapies see [Chia S.K., Speers C.H., D'yachkova Y. et al. The impact of new chemotherapeutic and hormone agents on survival in a population-based cohort of women with metastatic breast cancer. Cancer 2007; 110:973] and [Gennari A., Conte P., Rosso R. et al. Survival of metastatic breast carcinoma patients over a 20-year period: a retrospective analysis based on individual patient data from six consecutive studies. Cancer 2005; 104:1742] and [Dafni U., Grimani I., Xyrafas A. et al. Fifteen-year trends in metastatic breast cancer survival in Greece. Breast Cancer Res Treat 2010; 119:621].

The goals of treatment of metastatic breast cancer are to prolong survival and improve quality of life by reducing cancer-related symptoms. Cytotoxic chemotherapy (including the use of 5-FU) is particularly used in patients with hormone receptor-negative patients, patients with symptomatic hormone-receptor and a rapid disease progression or a large tumour burden involving visceral organs [Wilcken N., Hornbuckle J., Ghersi D.; Chemotherapy alone versus endocrine therapy alone for metastatic breast cancer. Cochrane Database Syst Rev 2003; :CD002747]. 5-FU is usually combined with cyclophosphamide and methotrexate (CMF). The reponse rate is around 20 % and the OS around 20 months
¹ Breast Cancer, http://www.cancerresearchuk.org/cancer-info/cancerstats/world/breast-cancer-world/ [Stockler M.R., Harvey V.J., Francis P.A. et al. Capecitabine versus classical cyclophosphamide, methotrexate, and fluorouracil as first-line chemotherapy for advanced breast cancer. J Clin Oncol 2011; 29:4498].

5-FU is also used for the treatment of advanced and recurring head and neck squamous cell cancer. The prognosis in this patient group is generally poor with a median survival time in most studies of 6-9 months. 5-FU is mainly used in combination therapies with platinum compounds. Response rates are around 30 % but the survival time remains low, around 6 months see [Clavel M., Vermorken J.B., Cognetti F. et al. Randomized comparison of cisplatin, methotrexate, bleomycin and vincristine (CABO) versus cisplatin and 5-fluorouracil (CF) versus cisplatin (C) in recurrent or metastatic squamous cell carcinoma of the head and neck. A phase III study of the EORTC Head and Neck Cancer Cooperative Group. Ann Oncol 1994; 5:521] and [Forastiere A.A., Metch B., Schuller D.E. et al. Randomized comparison of cisplatin plus fluorouracil and carboplatin plus 5-fluorouracil versus methotrexate in advanced squamous-cell carcinoma of the head and neck: a Southwest Oncology Group study. J Clin Oncol 1992; 10:1245].

But it is among the gastrointestinal tumours where the 5-FU based regimens have the widest use. Colorectal cancer (CRC) is the third most common cancer in men (10% of the total) and the second in women (9.2%), with over 1.3 Million cases (746 000 men and 614 000 women) reported worldwide during 2012. The geographic incidence of CRC varies widely across the world, and the geographical patterns are very similar in men and women. Incidence rates vary ten-fold in both sexes worldwide, the highest estimated rates being in Australia/New Zealand (ASR 44.8 and 32.2 per 100,000 in men and women respectively), and the lowest in Western Africa (4.5 and 3.8 per 100,000). The incidence increases with age and is highest amongst the elder population, i.e. 60-64 years: 67.4; 65-69 years: 95.1; 70-74 years: 127.8; and ≥ 75 years: 196.2 per 100 000 [Ferlay J, Soerjomataram I, Ervik M, Dikshit R, Eser S, Mathers C, Rebelo M, Parkin DM, Forman D, Bray, F. GLOBOCAN 2012 v1.0, Cancer Incidence and Mortality Worldwide: IARC CancerBase No. 11. Lyon, France: International Agency for Research on Cancer; 2013].

Approximately 40-50% of the affected patients develop metastatic disease and more than half a million deaths are reported annually as a consequence of CRC [Jemal A, Bray F, Center MM, Ferlay J, Ward E, Forman D. Global cancer statistics. CA Cancer J Clin. 2011 Mar-Apr;61(2):69-90]. Indeed CRC accounted for 694 000 deaths worldwide solely during 2012 (8.5% of the total) [Ferlay J, Soerjomataram I, Ervik M, Dikshit R, Eser S, Mathers C, Rebelo M, Parkin DM, Forman D, Bray, F. GLOBOCAN 2012 v1.0, Cancer Incidence and Mortality Worldwide: IARC CancerBase No. 11 [Internet]. Lyon, France: International Agency for Research on Cancer; 2013].

CRC patients are usually treated surgically and, in most circumstances, with curative intent. Surgery, in fact, remains the primary modality of treatment for malignancies of the lower gastrointestinal tract, and standard resection is the only therapy required for early-stage cancer [Nelson H, Petrelli N, Carlin A, Couture J, Fleshman J, Guillem J, et al. Guidelines 2000 for colon and rectal cancer surgery. J Natl Cancer Inst. 2001 Apr 18;93(8):583-96]. As the stage of the tumour advances, in terms of depth of penetration and lymph node involvement, the chance of cure with surgery alone diminishes and the rate of local recurrence increase. In such cases, surgery may either be combined with adjuvant treatment or be performed for palliative control of symptoms only.

Adjuvant therapies have been shown to improve treatment outcome in metastatic CRC with prolonged survival [Cunningham D, Atkin W, Lenz HJ, Lynch HT, Minsky B, Nordlinger B, et al. Colorectal cancer. Lancet. 2010 Mar 20;375(9719):1030-47]. Standard first-line adjuvant therapy of CRC includes single and combination chemotherapy with the agent 5-Fluorouracil (5-FU) [Cunningham D (2010)]. Treatment with 5-FU is usually given in combination with high doses of folate (or Leucovorin, LV) which significantly enhances the therapeutic effect of 5-FU in metastatic colorectal carcinoma. In fact, modulation of 5-FU with LV in metastatic disease has shown prolongation of the time-to-progression (TTP) of disease [Petrelli N, Douglass HO Jr, Herrera L, Russell D, Stablein DM, Bruckner HW, et al. The modulation of fluorouracil with leucovorin in metastatic colorectal carcinoma: a prospective randomized phase III trial. Gastrointestinal Tumor Study Group. J Clin Oncol. 1989 Oct;7(10):1419-26].

For colorectal tumors, the original response rate for 5-FU given as a monotherapy was only around 10%. By adding Leucovorin (LV) the response rate was improved to 21% [Thirion P, Michiels S, Pignon JP, Buyse M, Braud AC, Carlson RW, O'Connell M, Sargent P, Piedbois P (2004) Modulation of fluorouracil by leucovorin in patients with advanced colorectal cancer: an updated meta-analysis. J Clin Oncol 22(18):3766-3775]. However, LV needs to be converted to the active metabolite [6R]-5,10-methylenetetrahydrofolate (methyleneTHF), which subsequently forms a ternary complex with deoxyuridine monophosphate (dUMP) and the target enzyme thymidylate synthase (TS) in a reaction where dUMP is converted to dTMP [Jarmula A, Cieplak P, Montfort WR (2005) 5,10-Methylene-5,6,7,8-tetrahydrofolate conformational transitions upon binding to thymidylate synthase: molecular mechanics and continuum solvent studies. J Comput Aided Mol Des 19(2):123-136]. This reaction is inhibited when the fluorinated metabolite of 5-FU, FdUMP, binds the complex instead of dUMP [Parker WB, Cheng YC (1990) Metabolism and mechanism of action of 5-fluorouracil. Pharmacol Ther 48(3):381-395]. As such, LV does not have antitumoral effect, but enhances the effect of 5-FU by providing methyleneTHF in abundance, which stabilizes the ternary complex [Porcelli L, Assaraf YG, Azzariti A, Paradiso A, Jansen G, Peters GJ (2011) The impact of folate status on the efficacy of colorectal cancer treatment. Curr Drug Metab 12(10):975-984]. The inhibition impacts cells with a high proliferation rate most, such as tumor epithelial cells. This in turn leads to suppression of DNA synthesis in the cells, which may lead to cell death by apoptosis.

The required metabolic activation of LV into methyleneTHF is likely to lead to interindividual differences, which may be the reason the response rate for 5-FU given as a monotherapy was only improved to 21%.

A reduced folate, **fotrexorin calcium (CoFactor^{®})** ((*d*/)-5,10,-methylenepteroyl-monoglutamate calcium salt, or [6R,S]-5,10-methylene-THF Ca salt), also known as racemic methyleneTHF, has been suggested as an alternative to LV based on the assumption that direct administration of the reduced folate methyleneTHF in place of LV might offer significant advantages with respect to clinical activity. CoFactor^{®} is a 1:1 mixture of the two diastereoisomers [Odin, E., Carlsson, G., Frösing, R., Gustavsson, B., Spears, C.P., Larsson, P.A., 1998. Chemical stability and human plasma pharmacokinetics of reduced folates. Cancer Invest. 16, 447-455]. As the [6R]-isomer is the directly active co-substrate of TS, it was anticipated that administration of CoFactor^{®}, instead of leucovorin, would be advantageous due to lower inter- and intrapatient variability regarding both clinical safety and efficacy.

Indeed, in a Phase II Trial in previously untreated metastatic colorectal cancer, the response rate for CoFactor^{®} was found to be 35% [Saif, M.W, Merritt, J, Robbins J, Stewart J., Schupp, J, 2006. Phase III Multicenter Randomized Clinical Trial to Evaluate the Safety and Efficacy of CoFactor®/5-Fluorouracil/Bevacizumab Versus Leucovorin/5-Fluorouracil/ Bevacizumab as Initial Treatment for Metastatic Colorectal Carcinoma Clinical Colorectal Cancer, Vol. 6, No. 3, 229-234, 2006], and in another phase I/II clinical trial it was demonstrated that CoFactor^{®} combined with 5-FU showed clinical benefit in pancreas cancer, defined as stable disease or tumor response, in 40% of patients [Saif, M.W., Makrilia N., Syrigos K., 2010. CoFactor: Folate Requirement for Optimization of 5-Fluouracil Activity in Anticancer Chemotherapy. Journal of Oncology Vol. 1-5]. However, apart from presenting an unnecessary hepatic detoxification burden, the unnatural (6S)-isomer is a partial competitive inhibitor of the natural [6R]-isomer regarding its effect as co-substrate for TS [Leary, R.P., Gaumont, Y., Kisliuk, R.L., 1974. Effects of the diastereoisomers of methylenetetrahydrofolate on the reaction catalyzed by thymidylate synthetase. Biochem. Biophys. Res. Commun. 56, 484-488]. Furthermore, in a Phase IIb study CoFactor^{®} in colorectal cancer was not demonstrated to be more efficacious than leucovorin as no significant differences between the study arms with regard to either efficacy or safety could be found, and a planned Phase III study colorectal cancer was discontinued before completion [Press release: ADVENTRX Provides Update on Cofactor Program. Nov 2, 2007]. There thus remains a need for an improved folate-enhanced 5-FU treatment protocol.

### Definitions

As used herein, the terms IV or i.v. shall both mean **intravenous.**

As used herein, the term DLT shall refer to dose-limiting toxicity. Dose Limiting Toxicity (DLT) is a medical occurrence that is assessed as at least possibly related to a pharmaceutical product (i.e. to one or more chemotherapeutic agents) and is severe enough to prevent further increase in dosage or strength of treatment agent, or to prevent continuation of treatment at any dosage level.

As used herein, the term **ORR** shall refer to the Objective Response Rate, ie. the proportion of patients with reduction in tumor burden of a predefined amount. This shall be calculated as follows: ORR = Sum of partial responses plus complete responses as per RECIST 1.1 (a set of published rules that define when tumours in cancer patients progress during treatments, the responses being defined as:
- Complete Response: Disappearance of all target lesions.
- Partial Response: At least a 30% decrease in the sum of the diameters of target lesions, taking as reference the baseline sum diameters.
(Eisenhauer EA, Therasse P, Bogaerts J, Schwartz LH, Sargent D, Ford R, et al. New response evaluation criteria in solid tumours: revised RECIST guideline (version 1.1). Eur J Cancer. 2009 Jan; 45(2):228-47)

As used herein, the term **dU** shall refer to deoxyuridine.

As used herein, the term **BSA** refers to Body Surface Area

As used herein, the term **proliferative diseases** shall refer to a unifying concept that excessive proliferation of cells and turnover of cellular matrix contribute significantly to the pathogenesis of several diseases, including cancer, atherosclerosis, rheumatoid arthritis, psoriasis, idiopathic pulmonary fibrosis, scleroderma, cirrhosis of the liver, Crohn's disease and ulcerative colitis.

### STATEMENTS OF INVENTION

Recently a stable formulation of [6R]-5,10-methylenetetrahydrofolate ([6R]-5,10-MTHF) has been developed which is a stable formulation of the naturally occurring diastereoisomer of MTHF. As mentioned earlier, [6R]-MTHF is also a metabolite of Leucovorin (LV). Unlike LV, [6R]-MTHF, does not need to undergo further metabolism, and may be directly involved in the formation of the FdUMP-TS ternary complex.

According to the present invention, it has surprisingly been found that ORRs (objective response rates) of 50-60% can be achieved by treating colorectal cancer patients according to a regime whereby initial administration of 5-FU is followed by multiple IV boluses of [6R]-MTHF interspaced by an interval of about 10 min -4 hours between each bolus.

Accordingly, in **a first aspect** of the invention, [6R]-5,10-methylene-tetrahydrofolate is provided for use in a human in the treatment of solid tumors such as cancer, which treatment comprises the following steps:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² [6R]-5,10-methylenetetrahydrofolate, followed by
c) administering a continuous infusion of 5-FU (or an analog or prodrug thereof) over a period of 46 hours, or until the end of Day 2, followed by
d) optionally administering one IV bolus containing 5 - 1000 mg/m² (of BSA) [6R]-5,10-methylene-tetrahydrofolate before the end of Day 1, followed by
e) On Day 2, optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, and wherein step b) is optionally repeated up to 4 times on Day 1 with an interval of 10 min - 4 hours between each bolus being administered, and wherein step e) is optionally repeated up to 4 times on Day 2 with an interval of between 10 min - 4 hours between each bolus being administered, and wherein all steps a) - e) are optionally repeated every 2 weeks.

In a **second aspect** of the invention there is provided a method of treating a human diagnosed with a solid tumor such as cancer, which method comprises:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² (of BSA) [6R]-5,10-methylenetetrahydrofolate, followed by
c) administering a continuous infusion of 5-FU (or an analog or prodrug thereof) over a period of 46 hours, or until the end of Day 2, followed by
d) optionally administering one IV bolus containing 5 - 1000mg/m² (of BSA) [6R]-5,10-methylene-tetrahydrofolate before the end of Day 1, followed by
e) On Day 2, optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, and wherein step b) is optionally repeated up to 4 times on Day 1 with an interval of 10 min - 1 hours between each bolus being administered, and wherein step e) is optionally repeated up to 4 times on Day 2 with an interval of between 10 min - 4 hours between each bolus being administered, and wherein all steps a) - e) are optionally repeated every 2 weeks.

In a **third aspect** of the invention, [6R]-5,10-methylene-tetrahydrofolate is provided for use in a human in the treatment of solid tumors such as cancer, which treatment comprises the following steps:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² [6R]-5,10-methylenetetrahydrofolate, followed by
c) optionally administering one IV bolus containing 5 - 1000 mg/m² (of BSA) [6R]-5,10-methylene-tetrahydrofolate before the end of Day 1, followed by
d) On Day 2, administering an IV bolus containing 10-1000 mg/m² (of BSA) 5-FU (or an analog or prodrug thereof), followed, either simultaneously or after a period of 10 min-4 hours, by
e) optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, and wherein step b) is optionally repeated up to 4 times on Day 1 with an interval of 10 min - 4 hours between each bolus being administered, and wherein step d) is optionally repeated up to 4 times on Day 2 with an interval of between 10 min - 4 hours between each bolus being administered, and wherein all steps a) - e) are optionally repeated every 2 weeks.

In a **fourth aspect** of the invention, there is provided a method of treating a human diagnosed with a solid tumor such as cancer, which method comprises:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² [6R]-5,10-methylenetetrahydrofolate, followed by
c) optionally administering one IV bolus containing 5 - 1000 mg/m² (of BSA) [6R]-5,10-methylene-tetrahydrofolate before the end of Day 1, followed by
d) On Day 2, administering an IV bolus containing 10-1000 mg/m² (of BSA) 5-FU (or an analog or prodrug thereof), followed, either simultaneously or after a period of 10 min - 4 hours, by
e) optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, and wherein step b) is optionally repeated up to 4 times on Day 1 with an interval of 10 min - 4 hours between each bolus being administered, and wherein step d) is optionally repeated up to 4 times on Day 2 with an interval of between 10 min - 4 hours between each bolus being administered, and wherein all steps a) - e) are optionally repeated every 2 weeks.

### FIGURES

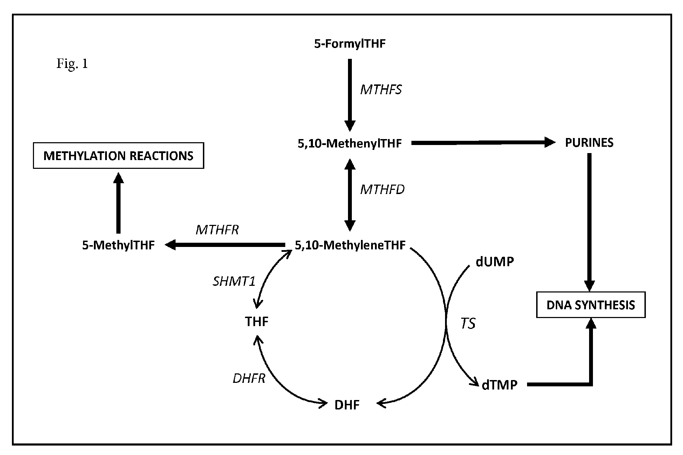

**Figure 1** (Wettergren Y, Taflin H, Odin E, Kodeda K, Derwinger K; Cancer Chemother Pharmacol (2015) 75:37-47) A simplified overview of the folate metabolism. Within the cells, [6R]-MTHF ([6R]-5,10-methyleneTHF) can be used directly as a methyl donor in the synthesis of dTMP from dUMP. The reaction is catalyzed by the enzyme thymidylate synthase (TS). Isovorin^{®} (levo-leucovorin, 5-formylTHF), on the other hand, needs to be converted in two steps to methyleneTHF. Treatment with 5-FU inhibits the synthesis of dTMP through the formation of FdUMP, which binds TS. DHF: dihydrofolate, DHFR: dihydrofolate reductase, SHMT1: serine hydroxymethyltransferase 1, MTHFR: methylenetetrahydrofolate reductase, MTHFD: methylenetetrahydrofolate dehydrogenase, MTHFS: methenyltetrahydrofolate synthetase.

### DETAILED DESCRIPTION OF THE INVENTION

5-Fluorouracil (5-FU) is possibly the most widely used anticancer drug in the world. It was discovered by Spears et al. (Spears et al., Cancer Res. 42:450 - 56 (1982)) that the therapeutic mechanism of 5-FU against murine colon cancer was complete inhibition of the DNA enzyme **thymidylate synthase** (TS) or abrogation of TS activity. As mentioned hereinabove, folates (specifically, tetrahydrofolates) serve as one-carbon donors in the synthesis of purines and the pyrimidine deoxythymidine monophosphate (dTMP) and can be used to modulate the action of 5-FU; see also Figure 1.

Several 5-FU based cancer treatment regimes have been developed where "folates" are given concomittantly or by other means as part of the treatment. Most of these regimes are variations over the FOLFOX regime which is the name of a combination chemotherapy treatment. It is also known as "Oxaliplatin de Gramont" or OxMdG, which means Oxaliplatin modified de Gramont. It is made up of the drugs:
FOL- Folinic acid (typically leucovorin or calcium folinate)
F-Fluorouracil (5-FU)
OX - Oxaliplatin

Examples of frequently administered chemotherapeutic agents within first-and second line metastatic CRC include 5-FU/folate, Capecitabine, Irinotecan, Oxaliplatin, Bevacizumab, Cetuximab, and Panitumamab, used alone or in combinations, e.g. FOLFOX (i.e. LV/ 5-FU/oxaliplatin), FOLFIRI (i.e. LV/5-FU/Irinotecan), FOLFOX/bevacizumab, and 5-FU-LV/bevacizumab and/or irinotecan.

As a specific example can be mentioned the **FOLFOX4** protocol, whereby 200 mg/m² Leucovorin is administered iv over 2 hrs before 5-FU on day 1 and day 2 (5-FU 400 mg/m² iv bolus and then 600 mg/m² iv continuous infusion over 22 hrs, day 1 and day 2. The protocol includes the administration of Oxaliplatin (Eloxatin) 85 mg/m² iv day 1, and the treatment is given Q2w x 12 cycles (see Goldberg RM et al. Pooled analysis of safety and efficacy of oxaliplatin plus 5-fluorouracil/leucovorin administrated bimonthly in elderly patients with colorectal cancer. J Clin Oncol 2006; 24:4085).

As another example can be mentioned the **ROSWELL PARK REGIMEN** whereby 5-FU is given as a 500 mg/m² BSA iv bolus 1 hour after starting the administration of a continuous infusion of leucovorin (500 mg/m² iv) over 2 hrs. This treatment is given Qw x 6 wks (once per week for six weeks) every 8 weeks for 3-4 cycles (see Lembersky BC et al. Oral uracil and tegafur plus leucovorin compared with iv 5-FU and leucovorin in stage II and III carcinoma of the colon: results from national surgical adjuvant breast and bowel project protocol C-06. J Clin Oncol 2006; 24:2059).

As yet another example can be mentioned the study design for the planned CoFactor^{®} Phase III study (Saif 2006, above), whereby CoFactor^{®} was to be administered at a dose of 60 mg/m² over 2-3 minutes by I.V. bolus followed 20 minutes later by the administration of 5-FU as a bolus over 2-3 minutes at a dose of 500 mg/m² each week for 6 weeks, repeated every 8 weeks. In this study, oxaliplatin was replaced by bevacizumab to be administered at a dose of 5 mg/kg as a continuous I.V. over 90 minutes every 2 weeks.

Typically, in currently employed treatment protocols such as the ones cited hereinabove, 5-FU is always administered after the folate adjuvant (eg Leucovorin), whereas by the present invention treatment is initiated by administering a bolus of 5-FU. As stated hereinabove, the highest response rates (ORRs) achieved employing such protocols have been on the order of 35-40%.

According to the present invention, it was therefore surprisingly found that ORRs (response rates) of 50-60% can be achieved by treating colorectal cancer patients according to a regime whereby initial administration of 5-FU is followed by multiple IV boluses of [6R]-MTHF interspaced by an interval of about 10 min - 4 hours between each bolus.

Accordingly, in **a first aspect** of the invention, [6R]-5,10-methylene-tetrahydrofolate is provided for use in the treatment in a human of a solid tumor such as cancer, which treatment comprises the following steps:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² [6R]-5,10-methylenetetrahydrofolate, followed by
c) administering a continuous infusion of 5-FU (or an analog or prodrug thereof) over a period of 46 hours, or until the end of Day 2, followed by
d) optionally administering one IV bolus containing 5 - 1000 mg/m² (of BSA) [6R]-5,10-methylene-tetrahydrofolate before the end of Day 1, followed by
e) On Day 2, optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, wherein step b) is optionally repeated up to 4 times on Day 1 with an interval of 10 min - 4 hours between each bolus being administered, and wherein step e) is optionally repeated up to 4 times on Day 2 with an interval of between 10 min - 4 hours between each bolus being administered, and wherein all steps a) - e) are optionally repeated every 2 weeks.

In an embodiment of the first aspect of the invention, step a) is preceded by administering an anticancer drug on Day 1, either as an IV bolus or as an infusion over a period of 1-4 hours.

In another embodiment [6R]-5,10-methylene-tetrahydrofolate is provided for the treatment according to the first aspect of the invention, wherein step a) is preceded by administering an anticancer drug on Day 1, either as an IV bolus or as an infusion over a period of 1-4 hours.

In a **second aspect** of the invention there is provided a method of treating a human diagnosed with a solid tumor such as cancer, which method comprises:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² (of BSA) [6R]-5,10-methylenetetrahydrofolate, followed by
c) administering a continuous infusion of 5-FU (or an analog or prodrug thereof) over a period of 46 hours, or until the end of Day 2, followed by
d) optionally administering one IV bolus containing 5 - 1000mg/m² (of BSA) [6R]-5,10-methylene-tetrahydrofolate before the end of Day 1, followed by
e) On Day 2, optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, wherein step b) is optionally repeated up to 4 times on Day 1 with an interval of 10 min - 1 hours between each bolus being administered, and wherein step e) is optionally repeated up to 4 times on Day 2 with an interval of between 10 min - 4 hours between each bolus being administered, and wherein all steps a) - e) are optionally repeated every 2 weeks.

In a **third aspect** of the invention, [6R]-5,10-methylene-tetrahydrofolate is provided for use in the treatment in a human of a solid tumor such as cancer, which treatment comprises the following steps:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² [6R]-5,10-methylenetetrahydrofolate, followed by
c) optionally administering one IV bolus containing 5 - 1000 mg/m² (of BSA) [6R]-5,10-methylene-tetrahydrofolate before the end of Day 1, followed by
d) On Day 2, administering an IV bolus containing 10-1000 mg/m² (of BSA) 5-FU (or an analog or prodrug thereof), followed, either simultaneously or after a period of 10 min - 4 hours, by
e) optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, wherein step b) is optionally repeated up to 4 times on Day 1 with an interval of 10 min - 4 hours between each bolus being administered, and wherein step d) is optionally repeated up to 4 times on Day 2 with an interval of between 10 min - 4 hours between each bolus being administered, and wherein all steps a) - e) are optionally repeated every 2 weeks.

In another embodiment, in the treatment according to the third aspect of the invention, step a) is preceded by administering an anticancer drug on Day 1, either as an IV bolus or as an infusion over a period of 1-4 hours.

In a **fourth aspect** of the invention, there is provided a method of treating a human diagnosed with a solid tumor such as cancer, which method comprises:
a) On Day 1, administering an IV bolus containing 10-1000 mg/m² (of BSA) 5-FU (or an analog or prodrug thereof), followed, either simultaneously or after a period of 10 min - 4 hours, by
b) administering one or more IV boluses, each containing 5 - 1000 mg/m² [6R]-5,10-methylenetetrahydrofolate, followed by
c) optionally administering one IV bolus containing 5 - 1000 mg/m² (of BSA) [6R]-5,10-methylene-tetrahydrofolate before the end of Day 1, followed by
d) On Day 2, administering an IV bolus containing 10-1000 mg/m² (of BSA) 5-FU (or an analog or prodrug thereof), followed, either simultaneously or after a period of 10 min - 4 hours, by
e) optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, wherein step b) is optionally repeated up to 4 times on Day 1 with an interval of 10 min - 4 hours between each bolus being administered, and wherein step d) is optionally repeated up to 4 times on Day 2 with an interval of between 10 min - 4 hours between each bolus being administered, and wherein all steps a) - e) are optionally repeated every 2 weeks.

In another embodiment of any of the aspects of the invention, step a) is preceded by administering one or more anticancer drugs on Day 1, either as an IV bolus or as an infusion over a period of 1-4 hours. In separate embodiments the anticancer drug may be one or more drugs selected from Platinum Drugs such as cisplatin (CDDP), carboplatin (CBDCA) and oxaliplatin (oloxetin), Antimetabolites such as 5-fluoruracil (5-FU), capecetabine (Xeloda), gemcitabine (Gemzar), methotrexate and pemetrexed (Alimta), Anti-tumor antibiotics, such as doxorubicin (Adriamycin), daunorubicin, actinomycin-D and mitomycin-C (MTC), Topoisomerase Inhibitors, such as irinotecan (CPT-11), topotecan (hycamtin) and etoposide (VP-16), Mitotic Inhibitors, such as paclitaxel (Taxol), docetaxel (Taxotere) and vincristine (Oncovin), Corticosteroids, such as prednisone, methylprednisolone (Solumedrol) and dexamethasone (Decadron), or may be selected from Targeted Therapies including Monoclonal Antibodies (MABs), such as cetuximab (Erbitux), rituximab (Rituxan) and bevacizumab (Avastin), or Small Molecular EGFR Inhibitors, such as gefitinib (Iressa), or may be selected from Hormone Therapies, such as tamoxifen (Nolvadex) and bicalutamide (Casodex), or may be slected from Cancer Immunotherapy Agents, including Monoclonal Antibodies, or Immune Check Point Inhibitors, such as PD-1 inhibitors including pembrolizumab (Keytruda) and nivolumab (Opdivo), or PD-L1 Inhibitors including atezolizumab (Tecentriq), or Cancer Vaccines.

In another embodiment of any of the aspects of the invention, the administered [6R]-5,10-methylene-tetrahydrofolate is a single diastereomer with a diastereomeric excess (d.e.) of >90% d.e., such as >93% d.e., such as >95% d.e., such as >98% d.e., such as >99% d.e., such as >99.5% d.e. or such as >99.9% d.e. In a preferred embodiment the administered [6R]-5,10-methylene-tetrahydrofolate is a single diastereomer with a diastereomeric excess (d.e.) of >98% d.e.

In another embodiment of any of the aspects the invention, the solid tumor is selected from various cancer forms including colon cancer, stomach cancer, breast cancer, bowel cancer, gallbladder cancer, lung cancer (specifically adenocarcinoma), colorectal cancer (CRC) including metastatic CRC, head and neck cancer, liver cancer and pancreatic cancer.

In a particular embodiment of any of the aspects the invention the solid tumor is selected from colon cancer and colorectal cancer.

In another embodiment of the invention, the 5-FU analog or prodrug is selected from fluorinated pyrimidine bases such as capecitabine (Xeloda), ie. N4-pentyloxycarbonyl-5'-deoxy-5-fluorocytidine, tegafur, 5-fluoro-pyrimidinone, UFT, doxifluridine, 2'-deoxy-5 fluorouridine, 5'-deoxy-5-fluorouridine, 1-(2'-oxopropyl)-5-FU, and alkyl-carbonyl-5-FU, BOF-A2, ftorafur(TS-1), and S-1.

In an embodiment [6R]-5,10-methylenetetrahydrofolate ([6R]-MTHF) is employed as a solid form which is soluble in water, such as a lyophilisate or a salt, optionally stabilized by one or more suitable excipients and/or antioxidants such as citric acid or ascorbic acid or salt forms thereof.

In an embodiment [6R]-5,10-methylenetetrahydrofolate ([6R]-MTHF) is administered as one or more IV boluses, each bolus containing 5 - 1000 mg/m² BSA, such as 5 mg/m² BSA, such as 7 mg/m² BSA, such as 10 mg/m² BSA, such as 15 mg/m² BSA, such as 30 mg/m² BSA, such as 60 mg/m² BSA, such as 120 mg/m² BSA, such as 240 mg/m² BSA, such as 480 mg/m² BSA, such as 720 mg/m² BSA or such as 960 mg/m² BSA.

In a further embodiment [6R]-5,10-methylenetetrahydrofolate is administered up to 4 times on Day 1 with an interval of 20-30 min between each bolus being administered.

In a another embodiment [6R]-5,10-methylenetetrahydrofolate is administered up to 4 times on Day 2 with an interval of 20-30 min between each bolus being administered.

In yet a another embodiment [6R]-5,10-methylenetetrahydrofolate is administered up to 4 times both on Day 1 and on Day 2 with an interval of 20-30 min between each bolus being administered.

In an embodiment 5-fluorouracil (5-FU) is administered as one or more IV boluses, each bolus containing 10-1000 mg/m² BSA, such as 300 mg/m² BSA, such as 400 mg/m² BSA, such as 500 mg/m² BSA, such as 600 mg/m² BSA, such as 700 mg/m² BSA, such as 800 mg/m² BSA, such as 900 mg/m² BSA or such as 1000 mg/m² BSA.

In an embodiment of any of the aspects of the invention, a treatment cycle comprises two days. This regimen may optionally be repeated every 2 weeks for four (4) cycles, i.e. a total of eight (8) weeks.

In another embodiment of any of the aspects of the invention, Day 1 and Day 2 of the treatment cycle are separated by a period of 1-5 days, for example for monitoring purposes.

In another embodiment of any of the aspects of the invention, the treatment cycle is extended beyond Day 1 and Day 2 by a period of 1-5 days.

### EXAMPLES

The safety and efficacy of [6R]-5,10-methylenetetrahydrofolate is analyzed in an open-label, multiple-site, Phase I/II Dose Cohort Trial in combination with a fixed dose of 5-Fluorouracil (5-FU) alone or together with a fixed dose of Oxaliplatin or Irinotecan in patients with stage IV colorectal cancer. A maximum of 63 Stage IV CRC patients eligible for 1st, 2nd or 3rd line treatment are planned to be enrolled in this study disposed as follows: three (3) to six (6) patients in each dose cohort, and three (3) additional patients in one dose cohort in each treatment arm according to the following study design (Table 1).

**TABLE 1: Initial Doses of the Chemotherapy Agents (Bevacizumab, Oxaliplatin, Irinotecan, and/or 5-FU) and of the Study Drug ([6R]-5,10-methylenetetrahydrofolate)**

| **Treament Arm** | **Cohort** | **Bevacizumab** | **Oxaliplatin^{¶}** | **Irinotecan^{#}** | **5-FU^{§}*** | **[6R]-5,10-methylenetetrahydrofolate** | **5-FU** |
|---|---|---|---|---|---|---|---|
| | | *At approx. -180 minutes (infusion 30 to 90 min)* | *At approx. -60 minutes (infusion 15 to 120 min)* | *At approx. -60 minutes (infusion 30 to 90 min)* | *At 0 minute (bolus)* | *At approx. 30 minutes (bolus)^{a}* | *At approx. 35 minutes (46-hour continuous infusion) ^{a}* |
| Arm 1 | Cohort 1 | N/A | N/A | N/A | 500 mg/m² | 30 mg/m² | N/A |
| | Cohort 2 | N/A | N/A | N/A | 500 mg/m² | 60 mg/m² | N/A |
| | Cohort 8 | N/A | N/A | N/A | 500 mg/m² | 120 mg/m² | N/A |
| | Cohort 9 | N/A | N/A | N/A | 500 mg/m² | 240 mg/m² | N/A |
| Arm 2 | Cohort 4 | N/A | 85 mg/m² | N/A | 500 mg/m² | 30 mg/m² | N/A |
| | Cohort 5 | N/A | 85 mg/m² | N/A | 500 mg/m² | 60 mg/m² | N/A |
| Arm 3 | Cohort 6 | N/A | N/A | 180 mg/m² | 500 mg/m² | 30 mg/m² | N/A |
| | Cohort 7 | N/A | N/A | 180 mg/m² | 500 mg/m² | 60 mg/m² | N/A |
| Arm 4 | Cohort 12 | N/A | 85 mg/m² | N/A | 400 mg/m² | 60 mg/m^{2 a} | 2 400 mg/m² |
| | Cohort 13 | N/A | 85 mg/m² | N/A | 400 mg/m² | 120 mg/m^{2 a} | 2 400 mg/m² |
| | Cohort 14 | N/A | 85 mg/m² | N/A | 400 mg/m² | 240 mg/m^{2 a} | 2 400 mg/m² |
| Arm 5 | Cohort 15 | 5 mg/kg | 85 mg/m² | N/A | 400 mg/m² | SP2D ^{a,b} | 2 400 mg/m² |

| | | | | | | | |
|---|---|---|---|---|---|---|---|
| Abbreviation: **N/A:** not applicable, **SP2D:** selected phase 2 dose. ¶ The time-point window for Oxaliplatin administration will be expanded to allow infusion times of up to 120 minutes, if necessary # The time-point window for Irinotecan administration will be expanded to allow infusion times of up to 90 minutes, if necessary. § The administered bolus 5-FU dose should not surpass the maximum recommended daily dose of 1000 mg, regardless of the body surface area. * Cohort #3, Cohort #10 and Cohort #11, originally included in earlier versions of this clinical study protocol, have been erased. a In Treatment Arm #4 (Cohorts #12, #13, and #14) and Arm #5 (Cohort #15) the total dose of ([6R]-5,10-methylenetetrahydrofolate will be divided into two (2) i.v. bolus injections dispensed approximately 30 and 60 minutes after administration of 5-FU bolus injection (at 0 minute), respectively. The continuous 5-FU infusion will be paused for administration of the second injection of ([6R]-5,10-methylenetetrahydrofolate. b The dose level of ([6R]-5,10-methylenetetrahydrofolate in Treatment Arm #4 (MOFOX) assessed as the dose level with the most favourable profile for the following investigation. | | | | | | | |

**[6R]-5,10-Methylenetetrahydrofolic acid** is formulated as a lyophylised powder containing 100 mg per vial (calculated as free acid). Dosing: Rapid i.v. bolus injections at a fixed dose of 30, 60, 120 or 240 mg/m², will be administered approximately 30 minutes after administration of 5-FU on Day 1 and Day 2 in each treatment cycle in all dose cohorts of the study (i.e. regardless of treatment arm). The regimen will be repeated every second week for four (4) cycles, i.e. eight (8) weeks.

**5-FU (5-fluorouracil)** is formulated as injection solution. Dosing: 5-FU will be administered as i.v. bolus injections on Day 1 and Day 2 in each treatment cycle. In Arm #2 and Arm #3 of the study, 5-FU will be administered approximately 60 minutes after start of Oxaliplatin or Irinotecan administration, respectively (see description below). The treatment will be repeated every second week for four (4) cycles, i.e. eight (8) weeks.

**Oxaliplatin** is formulated as a concentrated infusion solution. Dosing: Oxaliplatin will be administered as i.v. infusion during 15 - 120minutes on Day 1 in each treatment cycle in treatment Arm #2 of the study (i.e. Cohorts #4, #5, #10, and #11) and repeated every second week for four (4) cycles. Caution will be taken regarding toxicity associated with administration that may affect rate of infusion (e.g. grade ≤2 allergy, laryngopharyngeal dysesthesias, and laryngeal spasm). In such cases, rate of Oxaliplatin administration should be prolonged in following cycles according to clinical practice recommendations.

**Irinotecan** is formulated as a concentrated infusion solution. Dosing: Irinotecan will be administered as i.v. infusion during 30 - 90 minutes on Day 1 in each treatment cycle in treatment Arm #3 of the study (i.e. Cohorts #6 and #7) and repeated every second week for four (4) cycles. Caution will be taken regarding early toxicity (within 24 hours) associated with Irinotecan administration, i.e. acute cholinergic syndrome, characterized by early diarrhoea, emesis, diaphoresis, abdominal cramping, and, less commonly, hyperlacrimation and rhinorrhoea. In such cases, the use of anticholinergics according to clinical practice recommendations is necessary.

**Avastin** (Bevacizumab) is formulated as a concentrated infusion solution. Dosing: Bevacizumab is administered as i.v. infusion during 30-90 minutes on Day 1 in each treatment cycle in Treatment Arm #5 of the study (i.e. Cohorts #15) and repeated every second week for four (4) cycles.

Bevacizumab associated Toxicity: Based on data from clinical trials in which patients primarily were treated with Bevacizumab in combination with chemotherapy, the following may be recognized as Bevacizumab associated toxicity: Most common serious adverse events: gastrointestinal perforations, haemorrhage (including pulmonary haemorrhage / haemoptysis), and arterial thromboembolism; Most common adverse events: hypertension, fatigue or asthenia, diarrhoea, and abdominal pain.

## Claims

1. [6R]-5,10-methylene-tetrahydrofolate for the treatment in a human of solid tumors, including cancer, which treatment comprises the following steps:
a) On Day 1, administering an IV bolus containing 10-1000 mg/m² (of BSA) 5-FU (or an analog or prodrug thereof), followed, either simultaneously or after a period of 10 min - 4 hours, by
b) administering or more IV boluses, each containing 5 - 1000 mg/m² [6R]-5,10-methylenetetrahydrofolate, followed by
c) administering a continuous infusion of 5-FU (or an analog or prodrug thereof) over a period of 46 hours, or until the end of Day 2, followed by
d) optionally administering one IV bolus containing 5 - 1000 mg/m² (of BSA) [6R]-5,10-methylene-tetrahydrofolate before the end of Day 1, followed by
e) On Day 2, optionally administering one or more IV boluses each containing 5 - 1000 mg/m² (of BSA) [6R]-5,10-methylene-tetrahydrofolate,
wherein a total of at least two IV boluses [6R]-5,10-methylenetetrahydrofolate are administered before the end of Day 2, and wherein step b) is optionally repeated up to 4 times on Day 1 with an interval of 10 min - 4 hours between each bolus being administered, and wherein step e) is optionally repeated up to 4 times on Day 2 with an interval of between 10 min - 4 hours between each bolus being administered, and wherein all steps a) - e) are optionally repeated every 2 weeks.

2. The treatment of claim 1 wherein the solid tumor is a cancer form selected from colon cancer, stomach cancer, breast cancer, bowel cancer, gallbladder cancer, lung cancer (specifically adenocarcinoma), colorectal cancer (CRC) including metastatic CRC, head and neck cancer, liver cancer and pancreatic cancer.

3. The treatment of claim 1 or 2 wherein the solid tumor is a colorectal cancer (CRC) including metastatic CRC.

4. The treatment of any one of claim 1-3 where step a) is preceded by administering one or more anticancer drugs on Day 1, either as an IV bolus or as an infusion over a period of 1-4 hours.

5. [6R]-5,10-methylene-tetrahydrofolate for the treatment according to any one of claim 1-3, wherein step a) is preceded by administering one or more anticancer drugs on Day 1, either as an IV bolus or as an infusion over a period of 1-4 hours.

6. The treatment according to claim 4 or 5 wherein the anticancer drug is selected from oxaliplatin, irinotecan (CPT11) and bevacizumab (Avastin).

7. The treatment according to any one of the preceding claims wherein at least two boluses [6R]-5,10-methylenetetrahydrofolate are administered on Day 1 under step b).

8. The treatment according to any one of the preceding claims wherein at least two boluses [6R]-5,10-methylenetetrahydrofolate are administered on Day 2 under step e).

9. The treatment according to claim 7 wherein up to four boluses [6R]-5,10-methylenetetrahydrofolate are administered with an interval of 20-30 min between each bolus.

10. The treatment according to claim 8 wherein up to four boluses [6R]-5,10-methylenetetrahydrofolate are administered with an interval of 20-30 min between each bolus.

11. The treatment according to any one of the preceding claims wherein the [6R]-5,10-methylene-tetrahydrofolate is employed as a solid form which is soluble in water, such as a lyophilisate or a salt, optionally stabilized by one or more suitable excipients and/or antioxidants such as citric acid or ascorbic acid or salt forms thereof.

12. The treatment according to any one of the preceding claims wherein the [6R]-5,10-methylene-tetrahydrofolate has a diastereomeric purity of >98% d.e.
